# EUROPEAN PATENT SPECIFICATION

(11) **EP 2 318 513 B1**
(45) Date of publication and mention of the grant of the patent: **19.09.2012**
(21) Application number: 09780180.7
(22) Date of filing: 06.07.2009
(51) Int. Cl.: C12N 1/20, A61K 35/74, C12R 1/01

(54) **NEW PROBIOTIC BIFIDOBACTERIUM LONGUM**
NEUES PROBIOTISCHES BIFIDOBACTERIUM LONGUM
NOUVEAU BIFIDOBACTERIUM LONGUM PROBIOTIQUE

(30) Priority: 11.07.2008 DK 200800982
(43) Date of publication of application: 11.05.2011
(73) Proprietor: Chr. Hansen A/S, 2970 Hørsholm (DK)
(72) Inventor: KILDSGAARD, Jens, DK-2840 Holte (DK); LESER, Thomas Dyrmann, DK-1870 Frederiksberg (DK); GUNNARSSON, Thomas, S-21150 Malmö (SE); WEISE, Mette, DK-1958 Frederiksberg (DK); FOLKENBERG, Ditte Marie, DK-3400 Hilleroed (DK); JANZEN, Thomas, DK-2000 Frederiksberg (DK); FLAMBARD, Benedicte, DK-2000 Frederiksberg (DK)
(86) International application number: PCT/EP2009/058496
(87) International publication number: WO 2010/003916

(56) References cited:
- WO-A1-2007/093619
- US-B1- 6 306 638
- OUWEHAND ARTHUR C ET AL: "Bifidobacterium microbiota and parameters of immune function in elderly subjects." FEMS IMMUNOLOGY AND MEDICAL MICROBIOLOGY JUN 2008, vol. 53, no. 1, June 2008 (2008-06), pages 18-25, XP002552883 ISSN: 0928-8244
- SIGGERS RICHARD H ET AL: "Early administration of probiotics alters bacterial colonization and limits diet-induced gut dysfunction and severity of necrotizing enterocolitis in preterm pigs." THE JOURNAL OF NUTRITION AUG 2008, vol. 138, no. 8, August 2008 (2008-08), pages 1437-1444, XP002552884 ISSN: 1541-6100
- MATTARELLI P ET AL: "Proposal to reclassify the three biotypes of Bifidobacterium longum as three subspecies: Bifidobacterium longum subsp longum subsp nov., Bifidobacterium longum subsp infantis comb. nov and Bifidobacterium longum subsp suis comb. nov." INTERNATIONAL JOURNAL OF SYSTEMATIC AND EVOLUTIONARY MICROBIOLOGY, vol. 58, no. Part 4, April 2008 (2008-04), pages 767-772, XP002552885 ISSN: 1466-5026
- IBRAHIM S A ET AL: "Use of Chemical Mutagenesis for the Isolation of Food Grade beta-Galactosidase Overproducing Mutants of Bifidobacteria, Lactobacilli and Streptococcus thermophilus", JOURNAL OF DAIRY SCIENCE, AMERICAN DAIRY SCIENCE ASSOCIATION, US, vol. 83, no. 5, 1 May 2000 (2000-05-01), pages 923-930, XP027046086, ISSN: 0022-0302 [retrieved on 2000-05-01]
- DATABASE MEDLINE [Online] US NATIONAL LIBRARY OF MEDICINE (NLM), BETHESDA, MD, US October 2007 SÁNCHEZ BORJA ET AL: 'Low-pH adaptation and the acid tolerance response of Bifidobacterium longum biotype longum.' Database accession no. NLM17720838 & SÁNCHEZ BORJA ET AL: "Low-pH adaptation and the acid tolerance response of Bifidobacterium longum biotype longum.", APPLIED AND ENVIRONMENTAL MICROBIOLOGY OCT 2007 LNKD- PUBMED:17720838, vol. 73, no. 20, October 2007 (2007-10), pages 6450-6459, ISSN: 0099-2240
- G. Sermonti: "Mutation and Microbial Breeding" In: "Genetics of Industrial Microorganisms - Proceedings of the Third International Symposium on genetics of Industrial Microorganisms held at the University of Wisconsin Madison Wisconsin 4-9 June 1978", 1979, American Society for Microbiology pages 10-13,

## Description

### FIELD OF THE INVENTION:

The present invention relates to novel, probiotic, anti-inflammatory strains of *Bifidobacterium longum,* their use for prevention, alleviation or treatment of diseases, and for preparation of human or pet food, or pharmaceutical compositions.

### BACKGROUND OF THE INVENTION:

### Lactic acid bacteria

Bacteria which ferment sugars with the production of acids in particular lactic acid as a major metabolic component have been known for a long time. Such bacteria may be found in milk or milk products, living or decaying plants, but also in the intestine of man and animals. Traditionally, these bacteria have been referred to as "lactic acid bacteria". Lactic acid bacteria designates a rather heterologous group of Gram positive, non-motile, microaerophilic or anaerobic bacteria which ferment sugar with the production of acids, including lactic acid, and comprise e.g. the genera *Bifidobacterium, Enterococcus, Lactobacillus, Lactococcus, Leuconostoc and Pediococcus.*

For centuries lactic acid bacteria have been used in the manufacture of food and food products, including most dairy products, and today lactic acid bacteria are essential in the making of all fermented milk products such as yoghurt, soured whole milk, junket, cheese and butter.

The publication of a large amount of reports documenting that various lactic bacteria beneficially affect the well-being of humans and/or animals have attracted even further interest to this group of bacteria. In particular, specific strains of *Lactobacillus* or *Bifidobacterium* have been found to be able to colonize the intestinal mucosa and to assist in the maintenance of the well-being of the hosts. Such bacteria are commonly referred to as probiotic bacteria or probiotics.

### Probiotic bacteria

Probiotic microorganisms have been defined as "Live microorganisms which when administered in adequate amounts confer a health benefit on the host" (FAO/WHO 2002).

While a number of probiotic strains (in particular strains of *Lactobacillus* or *Bifidobacterium*) have been identified, numerous studies have also revealed that probiotic bacteria cannot accurately be predicted by reference to their taxonomic classification. Rather each strain should be assessed individually. Studies have also shown that even closely related probiotic strains may impose rather divergent effects on health.

It is however clear that most recognized probiotic strains, in addition to their health beneficial effect, possess a few extra characteristic features.

### Resistance to bile acids

It is generally considered a prerequisite for probiotics to exert their beneficial effects on the intestinal epithelium that adequate amounts of live probiotics are present. Upon entry into the upper part of the intestine the probiotic bacteria are subjected to high levels of bile salts. Thus it is of the uttermost importance that a probiotic bacterium is resistant to bile acids.

### Intestinal barrier function

Intestinal barrier function regulates transport and host defense mechanisms at the mucosal interface with the outside world. Transcellular and paracellular fluxes are tightly controlled by membrane pumps, ion channels and tight junctions, adapting permeability to physiological needs. Disturbance at any level, but particularly bacterial translocation due to increased permeability and breakdown of oral tolerance due to compromised epithelial and T cell interaction, can result in inflammation and tissue damage.

The invasion of high molecular weight substances such as LPS and other inflammatory compounds from the luminal side of the intestine into the circulating system is inhibited by the epithelial barrier.

One of the functions of this epithelial barrier is caused by the tight junctions. Tight junctions, or zonula occludens, are the closely associated areas of two epithelial cells whose membranes join together forming a virtual impermeable barrier to fluid, which separates the vascular system from the lumen of the digestive tract. Thus, a reduction of the tight junction barrier function has been demonstrated to result in an increased invasion of undesirable substances such as LPS from intestinal lumen into the circulating system. Conversely, induction of the tight junction barrier function is expected to result in a decreased invasion of undesirable substances such as LPS.

### Exopolysaccharides and biofilm formation

While survival is important for the adherence and colonization capacity (i.e. biofilm formation) it is also considered as an important contributing factor necessary for a probiotic bacterium to exert its beneficial effects whether it be via immune modulation, pathogen exclusion, or enhanced contact with the mucosa (1).

An important feature in biofilm development of many bacteria is a mucoid-like substance known as exopolysaccharides (EPS) or extracellular matrix. Exopolysaccharides are exocellular polymers present on the surface of many bacteria, including Lactobacilli and Bifidobacteria (2). EPS form a slime layer that is loosely attached to the cell surface or is secreted into the environment (3).

The physiological function of these molecules has been studied by comparing a non-EPS producing strain and an EPS producing isogenic variant. It was shown that the cell associated EPS protected the bacteria against bacteriophages and cell wall degrading enzymes (4). Recently, Mao *et al.,* found that the exopolysaccharides produced by an *Escherichia coli* strain enhance survival of the strain in simulated gastrointestinal fluids (5). These findings suggest that exopolysaccharides produced by certain bacteria may in fact be essential for their survival in the intestine.

A very important technical feature of a strain, which may be used in the dairy industry to prepare fermented milk products such as yoghurt, is its use in the improvement of the rheology and texture of fermented milk products, such as yoghurt (30). To a large extent the texture characteristics of a strain is linked up with the production of EPS (30). While this is an important technological feature certain EPS may, probably due to their specific chemical structure, impose beneficial health effects onto the recipients.

Some types of EPS produced by lactic acid bacteria have been suggested to have beneficial effects on human health such as cholesterol-lowering ability (6), immunomodulating activities (7;8), microbial adhesion to gastrointestinal mucus (9;10), antioxidant and free radical scavenging activities (11), and prebiotic (bifidogenic) effects (12).

Recently, a high level EPS producing *Lactobacillus delbrueckii* has been shown to significantly reduce the severity of inflammation in a murine model of colitis (13). Thus it seems clear that a high production of specific EPS may be related to the immunomodulatory effect of a probiotic strain.

### Gastrointestinal complications involving the gut microbiota

Inflammation is a complex reaction of the innate immune system that involves the accumulation and activation of leucocytes and plasma protein at sites of infection, toxin exposure or cell injury. Although inflammation serves as a protective function in controlling infections and promoting tissue repair, it can also cause tissue damage and disease. Gastrointestinal disorders such as inflammatory bowel disease (Crohn's disease), ulcerative colitis, and irritable bowel syndrome are idiopathic inflammatory conditions. Other diseases that relate to gastrointestinal inflammation are pouchitis, food allergies and atopic dermatitis resulting from food allergies (14-16).

Crohn's disease and ulcerative colitis are most prevalent in northern Europe, the UK and North America, where up to 500 per 100,000 are affected. While these diseases have different etiologies, all involve an inappropriate response of a malfunctioning mucosal immune system to the indigenous flora and other luminal antigens (6). Acute treatment of these disorders relies on antibiotics and anti-inflammatory drugs, and in severe cases surgery is necessary. Long term treatment includes lifestyle changes, dietary adjustments and smoking cessation (7). Probiotic bacteria have been shown to prolong remission from these conditions by exerting positive effects on the gastrointestinal epithelium and the mucosal immune system (8).

Irritable bowel syndrome (IBS) affects 7-31% of patients which have overcome infective gastroenteritis due to Salmonella, Campylobacter, or Shigella infection. IBS with diarrhea but without an infectious onset may occur in individuals experiencing "adverse" life events (17). Although clinical trials indicate large variations in the efficacy of probiotic bacteria in IBS, double blind, placebo controlled studies have shown that certain blends of probiotic strains may have beneficial effects on IBS (18-20), thus emphasizing the importance of identifying new probiotic strains.

### Immunomodulatory effects of probiotics

It has been shown that probiotic strains have the ability to alleviate the symptoms in patients with gastrointestinal inflammatory complications like Crohn's disease and ulcerative colitis (21;22). Specifically *Bifidobacteria* have been described as probiotic bacteria with potential in the prevention and treatment of gastrointestinal diseases as described in EP 1 688481, EP 0 199535, EP 0 768 375, WO 97/00078, EP 0 577 903 and WO 00/53200.

EP 0 768 375 (Nestle SA) describes specific strains of *Bifidobacterium* ssp, that are capable of becoming implanted in the intestinal flora and being capable of competitively excluding adhesion of pathogenic bacteria to intestinal cells. These *Bifidobacteria* are reported to assist in immunomodulation and thus in the maintenance of the individual's health. The immunomodulation effect of *Bifidobacteria* may even be conferred onto unborn children. WO 01/97822 e.g. describes that intake of *Bifidobacterium animalis* strain BB-12® by the mother during her pregnancy reduces the occurrence of atopic diseases in children. Also WO 03/099037 (Nestec SA) describes that the *Bifidobacterium animalis* strain BB-12® is able to beneficially modulate the immune response.

While quite a few *Bifidobacterium* strains with anti-inflammatory properties have been described, only a few strains have been reported to be effective in treating experimental induced intestinal inflammation *in vivo.*

WO04052462A1 (B. Pot, Danisco A/S) describes that certain *Bifidobacterium bifidum* and *Bifidobacterium lactis* strains are able to reduce the severity of inflammation in an experimental murine model of colitis.

WO2007/093619A1 (Nestec SA) describes that the *Bifidobacterium longum* strain BL999 (ATCC BAA-999) reduces the severity of inflammation in an experimental murine model of colitis.

EP 1688481 and EP 1141235 B (University College of Cork) describe that the *Bifidobacterium longum* infantis strain UCC35624 reduces the severity of inflammation in a murine model of colitis.

Foligne *et al.* (2007) describe three *Bifidobacteria* that reduce the severity of inflammation in the experimental murine model of colitis.

However, these documents are all silent with respect to the EPS production, the EPS composition of the strains, strength of tight junctions and, except for EP1688481, are also silent with respect to the bile acid resistance of the *Bifidobacterium* strains.

As even closely related probiotic strains impose rather divergent, but specific effects on health there is a constant need for new probiotic strains, including novel anti-inflammatory *Bifidobacterium* strains that are able to modulate *inflammatory intestinal diseases* are resistant to bile acids and able to colonize the intestine due to high levels of EPS.

### SUMMARY OF THE INVENTION:

The present inventors have surprisingly discovered that certain *B. longum* strains combine the important probiotic features of bile resistance, improvement of the Intestinal barrier function and a high production of exopolysaccharides with exceptional anti-inflammatory properties *in vivo*.

In particularly the present inventors have surprisingly identified one specific probiotic strain of *B. longum*, namely *Bifidobacterium longum* DSM 21062, which combines all the important probiotic features mentioned above.

It is contemplated that strains that are directly derived from this probiotic strain are likely to retain its probiotic features.

Thus, in a first aspect, the invention pertains to the strain of *Bifidobacterium longum* bacterial cells, which is useful as a probiotic, tolerates bile salts, produces a high amount of exopolysaccharide (EPS), and possesses anti-inflammatory effects in a trinitrobenzene sulfonate (TNBS)-induced colitis model in mice, characterized in that the strain is the *Bifidobacterium longum* strain with the registration number DSM 21062 or a mutant strain thereof, wherein the mutant strain is obtained by using the deposited strain as starting material, and wherein the mutant has retained or further improved the anti-inflammatory effects, the bile tolerance and/or the EPS expression that characterize DSM 21062.

As indicated in the examples the *Bifidobacterium longum* DSM 21062 strain possesses some extraordinary immonumodulation properties *in vivo,* indicating that it may be effective against a number of diseases. Thus, in a second aspect, the invention relates to the use of a composition comprising *Bifidobacterium longum* DSM 21062 bacterial cells or a mutant strain thereof, for the preparation of a medicament.

In particular the strain(s) may be used for the preparation of a medicament for the treatment of inflammatory conditions in the gastro-intestinal tract of a mammal.

Many probiotics are used for the manufacture of food or feed products; consequently a further important aspect of the invention is the provision of a human or pet food composition comprising *Bifidobacterium longum* DSM 21062 or a mutant strain thereof.

When preparing such food or feed products manufacturers usually make use of a so-called starter culture being cultures used to process food and feed products. Starter cultures are widely used in the diary industry. Typically starter cultures impart specific features to various food or feed products. It is a well established fact that the consistency, texture, body and mouth feel is strongly related to the EPS production of the starter culture used to prepare the food or feed. Thus a further aspect of the present invention is a starter culture composition comprising *Bifidobacterium longum* DSM 21062 or a mutant strain thereof, preferably wherein the starter culture composition is having a concentration of viable cells, which is in the range of 10⁴ to 10¹² CFU per gram of the composition.

The present invention also devices a method of manufacturing a food or feed product comprising adding a starter culture composition comprising *Bifidobacterium longum* DSM 21062 or a mutant strain thereof to a food or feed product starting material and keeping the thus inoculated starting material under conditions where the lactic acid bacterium is metabolically active.

As may be seen from the examples *Bifidobacterium longum* DSM 21062 not only produces high amounts of exopolysaccharides but also produces a rather unusual type of EPS being unusually rich in mannose and glucose residues.

It is well-known that some types of EPS relate to specific effects on human health (6), (9;10), (11), (12). Accordingly, it contemplated that the unusual structure of the EPS of *Bifidobacterium longum* DSM 21062 may be involved in, or even responsible for, the unique immunomodulation effects imposed by this strain.

It is further envisioned that the unique structure of the EPS of *Bifidobacterium longum* DSM 21062 may relate to inflammatory diseases in general. It has previously been shown that complex polymers containing mannose (mannans) possess significant biological activity when administered to mammals. Thus, one aspect of the invention relates to *Bifidobacterium longum* DSM 21062 cells or a fraction of said cells for use in the treatment of an inflammatory conditions in the gastro-intestinal tract of a mammal in general.

### DEFINITIONS

Prior to a discussion of the detailed embodiments of the invention a definition of specific terms related to the main aspects of the invention is provided.

As used herein the term "exopolysaccharide" designates a high-molecular-weight polymer that is composed of sugar residues that are secreted by a micro-organism into the surrounding environment.

By the expression "probiotics or probioticum" is referred a composition which comprises probiotic microorganisms. Probiotic bacteria are defined as microbial cells that have a beneficial effect on the health and well-being of the host. Probiotic microorganisms have been defined as "Live microorganisms which when administered in adequate amounts confer a health benefit on the host" (FAO/WHO 2002).

By the expression "prebiotic" is referred to a composition or a component of a composition which increases the number of probiotic bacteria in the intestine. Thus, prebiotics refers to any non-viable food component that is specifically fermented in the colon by indigenous bacteria thought to be of positive value, e.g. bifidobacteria, lactobacilli. The combined administration of a probiotic strain with one or more prebiotic compounds may enhance the growth of the administered probiotic *in vivo* resulting in a more pronounced health benefit, and is termed synbiotic.

By the expression "EPS which is relatively rich in mannose residues" is referred to an EPS in which mannose is at least the second most abundant type of monosaccharide present.

By the expression "Glu-N:mannose-ratio" is referred to the ratio between the amount of glucosamin (Glu-N) and mannose.

By the expression "EPS which is relatively rich in glucose residues" is referred to an EPS in which glucose is the most abundant type of monosaccharide present.

By the expression "Glu-N:glucose-ratio" is referred the ratio between the amount of glucosamin (Glu-N) and glucose.

Embodiments of the present invention are described below, by way of examples only.

### DETAILED DISCLOSURE OF THE INVENTION:

The invention aims at identifying a new probiotic strain which is useful for preventing, reducing or treating disorders, conditions or diseases associated with inflammation, in particularly inflammation of the intestine.

Here we, for the first time, describe probiotic *Bifidobacterium longum* strains which are characterized by tolerating bile salts, producing a high amount of exopolysaccharides (EPS), strengthening the light junctions of epithelial cells in vitro and possessing anti-inflammatory effects in a trinitrobenzene sulfonate (TNBS)-induced colitis model in mice.

In the preferred embodiment of the invention the *Bifidobacterium longum strain* is *Bifidobacterium longum* strain (DSM 21062).

The probiotic *Bifidobacferium longum* strain DSM 21062 was deposited on January 23, 2008 according to the Budapest Treaty on the International Recognition of the Deposit of Microorganisms for the Purposes of Patent Procedure with the Deutsche Sammlung von Mikroorganismen und Zellkulturen (DSMZ) Inhoffenstraße 7 B, 38124 Braunschweig, GERMANY.

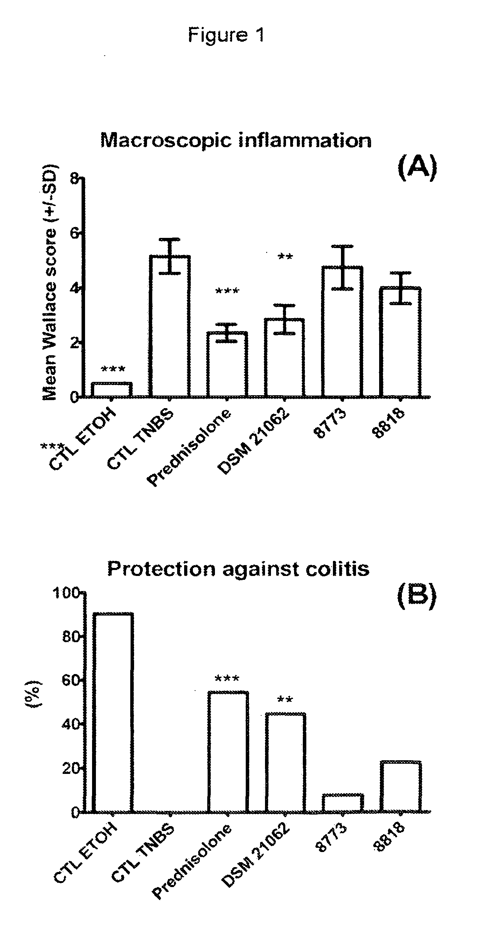

Example 1 demonstrates that *Bifidobacterium longum* strain (DSM 21062) is able to prevent inflammation in a mouse model of experimental colitis. As seen in figure fig. 1 the protective effect of *Bifidobacterium longum* strain (DSM 21062) on TNBS-induced colitis is clearly better than the effect of the other tested bacterial strains. The macroscopic inflammation score (Wallace score) of mice treated with DSM 21062 was 2.85 +/-0.52 while the score is higher for the other strains (strain 9555: 3.5 +/-0.53; strain 9553: 3-55 +/-0.58, strain: 8773: 4.75 +/-0.77, strain 8818: 4.0+/-0.58), The mean Wallace score for the TNBS reference was 5.15 +/-0.62 and the positive control of protection (prednisolone) was highly protective with a mean Wallace score of 2.35 +/-0.31, No signs of inflammation could be detected in healthy mice (Wallace scores of 0.5 were attributed all ethanol-treated mice).

The transepithelial electrical resistance (TEER) of a Caco-2 cell mono-layer enables the measurement of changes in paracellular ion flux linked to light junctional disruption (Velarde et al. (1999) Toxicology in Vitro 13, 723-727), and accordingly the assay can be used to quantify the protective effect of probiotic bacteria on the tight junctions. In example 6 it is demonstrated that the *Bifidobacterium longum* strain (DSM 21062) is superior to other probiotic strains with regard to its capacity to decrease the reduction of the transepithelial electrical resistance (TEER) of a Caco-2 cell layer in response to a challenge to tight junction disruptive sodium decanoate (C10). Without.being limited to theory, we contemplated that the tight junction strength is positive related to the intestinal barrier function and the tightness of the intestinal epithelium, and thus possibly related to the response of the epithelium when subjected to (TNBS)-induced colitis model in mice.

The *Bifidobacterium longum* strain (DSM 21062) furthermore produces high amount of EPS compared to the other bacterial strains in the study. The DSM 21062 produces at least 3 mg/ml, such as over 5 mg/ml or even 7 mg/ml or more in the EPS extract obtained as described in Example 2 and measured by HPLC on a PA10 column (high pH anion exchange chromatography).

However, not only the amount, but also the actual composition of the EPS is of importance for its function. It has previously been shown that complex polymers containing mannose (mannans) possess significant biological activity when administered to mammals. This includes activation of the immune system following the binding of mannose to recognition molecules such the mannose receptor (CD206) which is expressed on macrophages and dendritic cells (23-25). The mannose receptor has recently been suggested as a target for new vaccines, not only for mounting immune defenses against cancer and infectious diseases, but also for specific induction of tolerance in the treatment of autoimmune diseases (26). Intestinal dendritic cells (DCs) are believed to sample and present commensal bacteria to the gut-associated immune system (27) and suggest that EPS with a high content of mannose is able to modulate the immune response to the gut microflora. It has been shown that EPS from fungi possesses immunostimulatory and anti-tumor effects and that the EPS from these fungi contain mannose (28;29). To the best of our knowledge, it has not been suggested that the mechanism by which fungal EPS excerpt their immunostimulatory effect is by Interaction with the mannose binding receptor, we contemplate that it is the case. Consequently, it is considered an important feature of the *Bifidobacterium longum* strain (DSM 21062) of the present invention that it produces EPS which are relatively rich in mannose residues, e.g. measured as a Glu-N:Mannose-ratio of 40 or more, such as over 50 or even 60 or more. As illustrated in table II also the absolute amount of mannose residues is significantly higher that in the other bacteria investigated. Furthermore, the analysis of the EPS produced by DSM 21062 reveals that it is also surprisingly rich in glucose residues. The strain produces EPS which are relatively rich in glucose residues, e.g. measured as a Glu-N:Glucose-ratio of 50 or more, such as over 100 or even 180 or more. Also the absolute amount of glucose residues is surprisingly high.

It appears that the *Bifidobacterium longum* strain (DSM21062) produces EPS with a unique chemical composition. It is contemplated that these unique EPS are at least partly responsible for the beneficial effects of the strain. As mentioned, both the high amountof mannose residues and the amount of glucose residues of the EPS of the strain are considered noteworthy. We believe that in particular a polysaccharide preparation isolated from strain DSM 21062 (or a mutant thereof) and which is relatively rich both in mannose and glucose residues will prove particularly effective.

As illustrated in example 4 the *Bifidobacterium longum* strain (DSM 21062) is furthermore able to inhibit the growth of pathogenic bacterial strains. The example shows that this strain is able to inhibit bacterial strains selected from the group consisting of *Listeria monocytogenes, Salmonella ssp; Staphylococcus aureus and Escherichia coli.*

It is noteworthy that the *Bifidobacterium longum* strain (DSM21062) is able to inhibit the growth of pathogenic bacterial strains since production of antimicrobial substances other than organic acids is uncommon for Bifidobacterium strains. Only a few strains have been isolated, which were able to produce hydrogen peroxide and heat-stable proteinaceous antimicrobial compounds (30). EP 1688481 (Univ. College of Cork) describes a *Bifidobacterium longum infantis* strain with antimicrobial activity. The antimicrobial activity of this strain is possibly due to the production of acetic and lactic acids as the assay performed did not involve pH adjustment. We have developed an assay that evaluates the antimicrobial activity of pH adjusted conditioned medium from probiotic strains *Bifidobacterium longum* DSM 21062, CHCC9555, CHCC9553, CHCC8773 and CHCC8818. In this assay *Bifidobacterium longum* DSM 21062 is clearly superior at inhibiting pathogen growth and this effect is, as opposed to the *Bifidobacterium longum infantis* strain described in EP 168848, not due to production of acetic or lactic acid, and indicates that *Bifidobacterium longum* DSM 21062 exerts its antimicrobial effects via a unique mechanism.

The TNBS-induced colitis resulted in a significant weight loss in the mice which could be counteracted to a certain extent by DSM 21062 but not by the other bacterial treatments. Thus in one embodiment the strain(s) of the invention are characterized by that animals receiving the strain experience a weight loss due to TNBS-induced colitis that is significantly less (at the p=0.1 level) than the weight loss in animals receiving control strains.

It is clear from the above that DSM 21062 can be useful for the preparation of a medicament. Example 1 shows that DSM 21062 can be used for the preparation of a medicament for the treatment of inflammatory conditions in the gastro-intestinal tract of a mammal. Examples of inflammatory conditions in the gastro-intestinal tract of man include irritable bowel syndrome (IBS), inflammatory bowel disease (IBD), celiac disease (lactose intolerance), Crohn's disease, interstitial cystitis, acidic gut syndrome, gastritis, ulcerative colitis, diarrhea, typhus and intestinal inflammation associated with food allergies. In one embodiment of the present invention DSM 21062 is used for the preparation of a medicament directed towards the prevention, alleviation or treatment of any of these conditions.

In a further aspect, the present invention relates to a human or pet food composition comprising DSM 21062 or a mutant strain thereof. Preferably, the bacteria may be administered as a supplement to the normal diet or as a component of a nutritionally complete human or pet food. The dosage form may be liquid or solid. In the latter case, the product may be powdered and formed into tablets, granules or capsules or simply mixed with other food ingredients to form a functional food.

The food composition of the present invention can be any ingestible material selected from the group consisting of milk, curd, milk based fermented products, acidified milk, yoghurt, frozen yoghurt, milk powder, milk based powders, milk concentrate, cheese, cheese spreads, dressings, beverages, ice-creams, fermented cereal based products, infant formulae, tablets, liquid bacterial suspensions, dried oral supplement, wet oral supplement, dry tube feeding or wet tube feeding that is produced by use of the DSM 21062 or a mutant strain thereof.

In a further embodiment, the composition further comprises a pharmaceutically acceptable carrier. As used herein, the term "pharmaceutically acceptable carrier" means one or more solid or liquid filler diluents or encapsulating substances which are suitable for administration to a human or an animal and which is/are compatible with the probiotically active organisms. The term "compatible" relates to components of the pharmaceutical composition which are capable of being commingled with the DSM 21062 or a mutant strain thereof in a manner enabling no interaction that would substantially reduce the probiotic efficacy of the organisms selected for the invention under ordinary use conditions. Pharmaceutically acceptable carriers must be of a sufficiently high purity and a sufficiently low toxicity to render them suitable for administration to humans and animals being treated.

A solid composition as described herein is preferably a tablet, a capsule or a granulate (comprising a number of granules). Preferably the solid composition is an oral dosage form. A review of conventional formulation techniques can be found in e.g. "The Theory and Practice of Industrial Pharmacy" (31) or (32). Thus, the tablets may be prepared by methods known in the art and can be compressed, enterically coated, sugar coated, film coated or multiply compressed, containing suitable binders, lubricants, diluents, disintegrating agents, coloring agents, flouring agents, flow-inducing agents and melting agents. Capsules, both soft and hard capsules, having liquid or solid contents, may be prepared according to conventional techniques that are well known in the pharmaceutical industry. As one example, the probiotically active organisms may be filled into gelatine capsules, using a suitable filling machine. A solid composition as described herein may also be a pellet.

The human or pet food composition or dosage form should comprise at least DSM 21062 or a mutant strain thereof, as described above, so that the amount of each of the two strains that is available for the individual is of about 10³-10¹⁴ CFU per day, such as 10⁶-10¹³ CFU per day including 10⁸-10¹² CFU per day or even 10⁹-10¹¹ CFU per day. This amount depends on the individual weight, and it is preferably of about 10⁹-10¹² CFU /day for humans and 10⁷-10¹⁰ CFU/day for pets. It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy.

In a further embodiment the human or pet food composition or dietary supplement dosage form further comprise one or more prebiotic substances. Examples of suitable prebiotic substances are fructo-oligosaccharides (FOS) and inulin. However other prebiotic substances such as galacto-oligosaccharides (GOS), mannan-oligosaccharides (MOS) and even a polysaccharide composition obtained from the bacterial cells of DSM 21062 are also contemplated.

Microorganisms are involved in the manufacture of food and feed products including most dairy products. Bacterial cultures, in particular cultures of bacteria generally classified as lactic acid bacteria, are essential in the making of all fermented milk products, cheese and butter. Cultures of these microorganisms are often referred to as starter cultures and impart specific features to various dairy products by performing a number of functions. In order for the starter culture to exert its function it is essential that it comprises live cells in sufficient amounts. Thus one embodiment of the present invention is a starter culture composition comprising living B. *longum* DSM 21062 or a mutant strain thereof, and preferably wherein the starter culture composition is having a concentration of viable cells, which is in the range of 10⁴ to 10¹² CFU per gram of the composition.

Starter cultures are typically used for the manufacturing a food or feed product by adding the starter culture composition according to a food or feed product starting material and keeping the thus inoculated starting material under conditions where the lactic acid bacterium is metabolically active. In a preferred embodiment the food product is a milk-based product such as cheese, yoghurt, butter or a liquid fermented milk product, such as e.g. buttermilk or drinking yoghurt. The use of the present invention for the manufacturing of cow milk products is especially preferred.

While the probiotic effect is related to live cells a number of reports have revealed that also dead or inactivated bacteria may posses unique health beneficial properties. Accordingly, one embodiment relate to dead or inactivated B. *longum* DSM 21062 or a mutant strain thereof or a fraction of said cells for use in treatment of an inflammatory condition in the gastro-intestinal tract of a mammal. In particular such cells or fractions of such cells are used for the preparation of a medicament for the treatment of inflammatory conditions in the gastro-intestinal tract of a mammal such as irritable bowel syndrome (IBS), inflammatory bowel disease (IBD), celiac disease (lactose intolerance), intestinal inflammation associated with food allergies, Crohn's disease, interstitial cystitis, acidic gut syndrome, gastritis, ulcerative colitis, diarrhea or typhus.

The invention is further illustrated in the following non-limiting examples and figures wherein:
Figure 1: (A) Macroscopic inflammation evaluation of the TNBS-induced colitis expressed as Wallace score. Mice treated with bacteria are compared to mice receiving TNBS or sham treatment alone. (B) Macroscopic inflammation evaluation of the TNBS-induced colitis expressed as % protection compared to mice receiving TNBS or sham treatment alone. * identifies that the protection is significantly different from the sham treatment at the p ≤ 0.05 level , ** a statistical significance level of p ≤ 0.01, *** a statistical significance level of p ≤ 0.002, (*) not significant (t-test). Error bar indicate 1 x standard error of the mean.

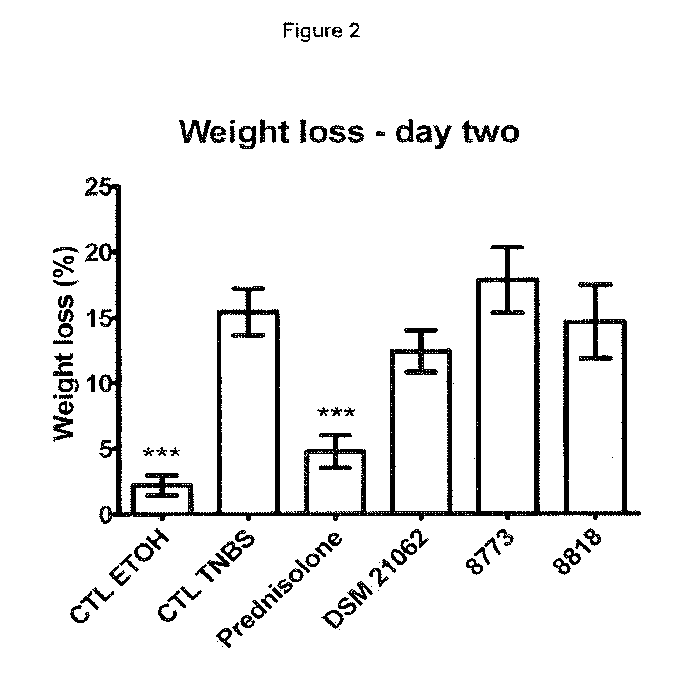

Figure 2: None of the bacterial treatments could reduce weight loss, except for the strain DSM 21062 where a positive trend in reduction of body mass loss was noted (12.4% +/-1.6, p = 0.09). The positive control of protection (prednisolone) was highly significant in terms of reducing weight loss (4.75% +/- 1.25, p ≤ 0.001). *** identifies that the protection is significant different from the sham treatment at the p ≤ 0.001 level.

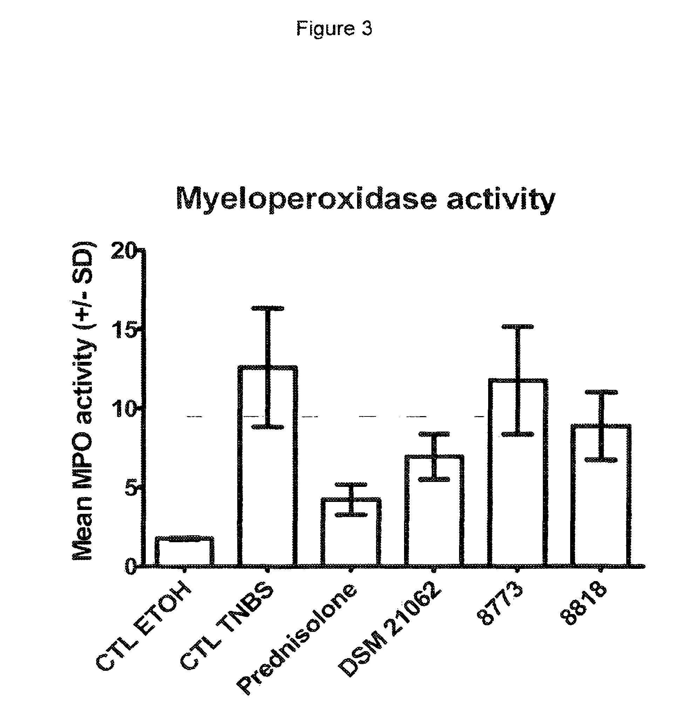

Figure 3: The MPO assay confirmed the massive infiltration of neutrophils in inflamed tissues, especially for the TNBS control group (12.56 +/- 3.75), in contrast to the "healthy mice" where very weak activities were measured (1.75 +/- 0.07). The protection by the corticoid control significantly reduced this parameter (4.21 +/-0.96). Mean values for all bacteria treated groups were all found to be well under the values of the positive control, however no significant reductions were observed. The strain exhibiting the best reduction was strain DSM 21062 (p = 0.07). Error bar indicate 1 x standard error of the mean.

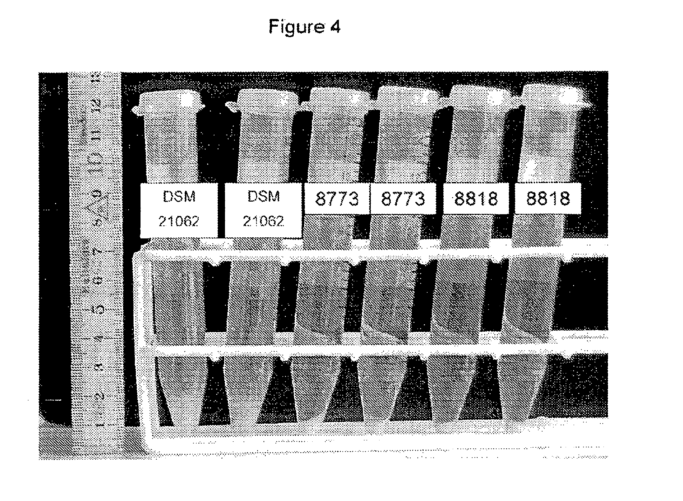

Figure 4: The bulkiness of *Bifidobacterium longum* DSM 21062, the *Lactobacillus ruminis* strain CHCC8818 and the *Lactobacillus paracasei* strain CHCC8773 illustrated by the pellet size formed when equal amounts of bacterial biomass are sedimented.

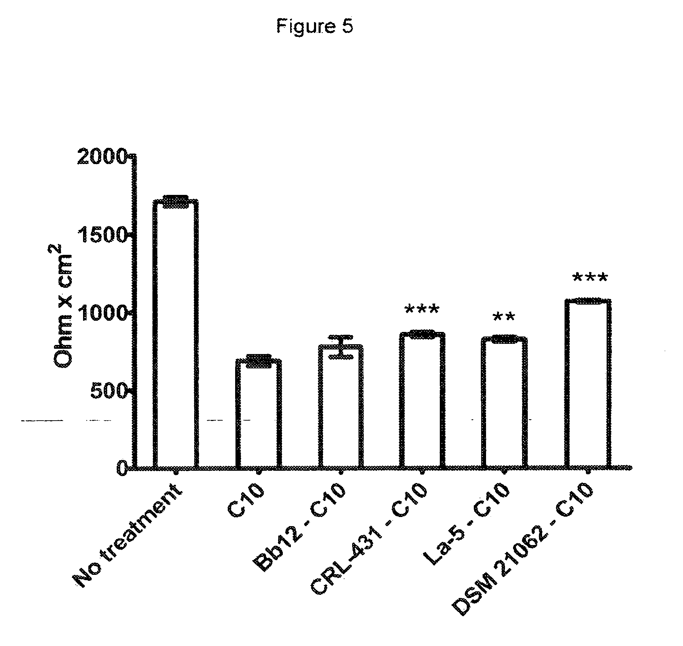

Figure 5: Measurements of transepithelial electrical resistance across confluent layer of Caco-2 cells. "No treatment" is Caco-2 cell layers treated with neither C10 nor probiotics and "C10" is cell layers treated with C10 only. Probiotic treatment (Bb-12, CRL-431, La-5 and DSM 21062) of the cell layers followed by a C10 challenge are indicated in the four right columns. ). Error bar indicate 1 x standard error of the mean. ** identifies a statistical significance level of p≤0.01, *** a statistical significance level of p ≤ 0.001 (t-test).

### EXAMPLES:

### Example 1: In vivo analysis of anti-inflammatory potential of Bifidobacterium longum DSM 21062

### Culture conditions and strains used

In the present study the following strains were used: *Bifidobacterium longum* strain DSM 21062 (CHCC8879). The strains *Lactobacillus ruminis* strain CHCC8818, *Lactobacillus paracasei* strain CHCC8773, *Bifidobacterium bifidum* strain CHCC9555 and *Bifidobacterium bifidum* strain CHCC9553 were used a control strains. Strain CHCC8818, CHCC8773, CHCC9555 and CHCC9553 are 4 putative probiotic strains of the Chr. Hansen culture collection identified and studied in the screening procedure resulting in the present invention.

*Lactobacilli* were grown in MRS broth (Difco, BD Diagnostics, Sparks, MD, USA) at 37°C.

*Bifidobacteria* were grown at 37°C in MRS broth + 0.05 % hydrochloride cysteine, using Anaerocult A incubation bags (Merck, Darmstadt, Germany).

For each strain, the OD 600 nm/CFU (colony forming units) correspondence has been established on the basis of growth curves and bacterial enumeration on agar medium.

Concerning the animal experiment, 500 µl of each strain was inoculated daily from distinct stock vials into 50 ml of the corresponding growth medium to ensure a final bacterial count of > 10¹⁰ CFU.

Cultures were centrifuged, washed in PBS and resuspended in an adequate volume of buffer before the bacteria are administered to the animals.

### TNBS induced colitis and inflammation scoring

The protocols describing the model and the assays for inflammatory markers correspond to a standardized methodology, previously described in (33). Briefly, groups of 10 mice were given either carbonate buffer ("TN BS-positive control") or freshly cultivated 1 x 10⁹ live bacteria daily ("treatment") for five consecutive days via the intragastric route. Additionally, a group of 10 mice was treated with a commercial preparation of prednisone (Cortancyl, Sanofi Aventis, France) at 10 mg/kg, administered orally 5 days prior to TNBS administration and for 2 subsequent days after the TNBS treatment ("Positive protection control").

Acute colitis was triggered on day 5 by intra-rectal administration of a 50 µl solution of TNBS (Sigma-Aldrich Chemical) in 50% ethanol. An administration of 100 mg/kg TNBS yielded an intestinal inflammation of medium severity in the BALB/c mice used. A group of 10 mice was also given carbonate buffer but received 50 µl of a 50% ethanol solution only (no TNBS; "negative (healthy) control"). Animals were subsequently monitored daily for loss of body weight. Two days after induction of colitis, the mice were sacrificed. The study design is shown in Table I. After mouse dissection, two independent observers blindly scored the macroscopic inflammation of the colon on the Wallace scale (34). The % relative protection was calculated as 100 x (average Wallace score "TNBS-positive control group - average Wallace score "treatment" group) / average Wallace score "TNBS-positive control group as previously described.

**Table I: Study design of in vivo TNBS-induced colitis. (i.r. = intrarectal administration, i.g.=intragastric administration)**

| **N=10 mice/group** | **Day -4** | **Day -3** | **Day -2** | **Day -1** | **Day 0** | **Day 2** |
|---|---|---|---|---|---|---|
| PBS without colitis (healthy mice) | i.g. | i.g. | i.g. | i.g. | Intragastric administration + 50% ETOH i.r. | Macroscopic score + weight loss |
| PBS colitis control (sick mice) | i.g. | i.g. | i.g. | i.g. | Intragastric administration + TNBS i.r. | Macroscopic score + weight loss |
| Treatment control: Prednisolone 0.2 mg/day/mouse | i.g. | i.g. | i.g. | i.g. | Intragastric administration + TNBS i.r. | Macroscopic score + weight loss |
| CHCC9555: 10⁹ CFU/day/mouse | i.g. | i.g. | i.g. | i.g. | Intragastric administration + TNBS i.r. | Macroscopic score + weight loss |
| CHCC9553: 10⁹ CFU/day/mouse | i.g. | i.g. | i.g. | i.g. | Intragastric administration + TNBS i.r. | Macroscopic score + weight loss |
| DSM21062: 10⁹ CFU/day/mouse | i.g. | i.g. | i.g. | i.g. | Intragastric administration + TNBS i.r. | Macroscopic score + weight loss |
| CHCC8773: 10⁹ CFU/day/mouse | i.g. | i.g. | i.g. | i.g. | Intragastric administration + TNBS i.r. | Macroscopic score + weight loss |
| CHCC8818: 10⁹ CFU/day/mouse | i.g. | i.g. | i.g. | i.g. | Intragastric administration + TNBS i.r. | Macroscopic score + weight loss |

### Myeloperoxidase assay

The activity of the enzyme MPO, a marker of polymorphonuclear neutrophil primary granules, was measured in proximal colon tissue according to Bradley et al, 1982 (35). Immediately after sacrifice, a colonic sample (1 cm long) was taken at 3 cm from the ceco-colonic junction. Samples were suspended in a potassium phosphate buffer (50 mmol/L, pH 6.0) and homogenized in ice using a polytron. Three cycles of freezing and thawing were undertaken. Suspensions were then centrifuged at 10,000 g for 15 min at 4°C. Supernatants were discarded and pellets were resuspended in hexadecyltrimethylammonium bromide buffer (HTAB 0.5%, w/v, in 50 mmol/L potassium phosphate buffer, pH 6.0), a detergent inducing release of MPO from the polymorphonuclear neutrophil primary granules. These suspensions were sonicated on ice, and again centrifuged for 15 min at 4°C. Supernatants obtained were diluted in potassium phosphate buffer (pH 6.0) containing 0.167 mg/mL of O-dianisidine-dihydrochloride and 0.0005% of hydrogen peroxide (H2O2). MPO from human neutrophils (0.1 U/100 mL, Sigma) was used as a standard. Changes in absorbance at 450 nm, over 5 and 10 min, were recorded with a microplate spectrophotometer. MPO activity was expressed as International Units of MPO/cm of intestine. One unit of MPO activity was defined as the quantity of MPO degrading 1mmol hydrogen peroxide/min/mL at 25°C.

### Statistical analysis

Results were analyzed by the non-parametric one-way analysis of variance, Mann-Whitney U test (XLSTAT software: http://www.xlstat.com). Differences were judged to be statistically significant when the p value was < 0.05.

### Results

The TNBS-induced inflammation resulted in colitis with medium severity, leading to a mean Wallace score of 5.15 for the TNBS reference, while no signs of inflammation could be detected in healthy mice (Wallace scores of 0.5 were attributed for all ethanol -treated mice). The positive control of protection (prednisolone) was highly protective with a mean Wallace score of 2.35 (corresponding to 54.4% of protection, p = 0.002) (see figure 1A and B).

The *Bifidobacterium longum* strain DSM 21062 (CHCC8879) protects from TNBS-induced injury by 44% (p = 0.007), clearly superior to the other *Bifidobacteria* strains (CHCC9555; CHCC9553) which protect by 32 and 31 %, respectively.

The strains CHCC8773 and CHCC8818 did not lead to significant protection (7.76% and 22.3%, respectively).

The induced colitis (100 mg/kg of TNBS) can be defined as of medium severity, with no mortality and a body weight loss of 15.4% +/- 1.77% for the positive reference group. The loss in body weight two days after colitis induction is shown in figure 2.

None of the bacterial treatments could reduce weight loss, except for the strain DSM 21062 where a positive trend in reduction of body mass loss was noted (12.4% +/- 1.6, p = 0.09). The positive control of protection (prednisolone) was highly significant in terms of reducing weight loss (4.75% +/- 1.25, p< 0.001).

The measurements of MPO activity are shown in figure 3. The MPO assay confirmed the massive infiltration of neutrophils in inflamed tissues, especially for the TNBS control group (12.56 +/- 3.75), in contrast to the "healthy mice" where very weak activities were measured (1.75 +/- 0.07). The protection by the corticoid control significantly reduced this parameter (4.21 +/- 0.96).

Mean values for all bacteria treated groups were all found to be well under the values of the positive control, however no significant reductions were observed. The strain exhibiting the best reduction was strain DSM 21062 (p = 0.07).

### Example 2: EPS purification, quantification and composition of the strains

### Culture conditions

*Lactobacilli* were grown 24-48 hrs in 200 ml MRS at 37°C. *Bifidobacteria* were grown at 37°C in 200 ml MRS + 0.05% hydrochloride cysteine, using Anaerocult A incubation bags (Merck) for 24-48 hrs.

### EPS purification

EPS purification was carried out essentially as described in (36). Briefly, after growth of 200 ml bacterial cultures, trichloroacetic acid was added to the cultures to a final concentration of 4%. Cells and protein were removed by centrifugation and the supernatant passed through a 0.2 µm filter. The EPS were precipitated by addition of an equal volume of ice cold ethanol. The precipitate was collected by centrifugation and the EPS re-dissolved in purified water.

### Monosaccharide composition and concentration

The analysis of the monosaccharide composition of the purified EPS was performed by high pH anion exchange chromatography (HPAEC) as described by (3). Briefly, purified EPS solutions were mixed with 4M TFA (50% vol/vol) and hydrolysed for 2 hours at 100°C. The reaction was stopped by cooling in icewater for 30 min. Samples were dried using nitrogen flush and subsequently resuspended in ddH2O to the initial volume. The applied HPAEC method provided the concentration (in ppm) of glucose, galactose, fucose, rhamnose, glucose-amine, galactose-amine, mannose and glucoronic acid using fructose as internal standard. The stationary phase was a Carpopac (PA10) column designed for quantitative determination of mono- and disaccharides. An amino trap column was placed before the Carbopac column in order to retain amino acids from the samples, and a borate trap column was placed in the eluent stream before the injection valve. All equipment was from Dionex Corporation. The mobile phase consisted of the three eluents: NaOH (200 mM), NaAc (300mM) and ddH2O applied in a gradient.

Concentration of the monosaccharides was calculated on basis of the response of the analyte relative to the response of the internal standard (fructose) added to the samples and standards during sample preparation.

### Results

The analysis of the monosaccharide composition of the EPS purified from individual strains indicated that the strain DSM 21062 is characterized by the expression of a high concentration of EPS (7.7 mg/ml) compared to the other strains which produce less than 1.97 mg/ml. Furthermore, the EPS from DSM 21062 are uniquely characterized by glucose and mannose levels that are 10- and 1.8-fold higher than the other strains, respectively (see table II).

**Table II: The concentration and composition of the purified EPS as measured by HPLC on a PA10 column (high pH anion exchange chromatography). (Fuc: Fucose, Rham: Rhamnose, Glu-n: Glucosamin, Gal-N: Galactosamin, Gal: Galactose, Man: Mannose, H-Glu: Glucoronic acid).**

| **Strain** | **Fuc (ppm)** | **Rham (ppm)** | **Glu-N (ppm)** | **Gal-N (ppm)** | **Gal (ppm)** | **Glu (ppm)** | **Man (ppm)** | **H-Gluc (ppm)** | **Concentration (mg/ml)** |
|---|---|---|---|---|---|---|---|---|---|
| CHCC8773 | 44 | nd | 136 | 42 | 121 | 474 | 1008 | 146 | 1,97 |
| CHCC8818 | 21 | nd | 80 | 21 | 59 | 366 | 762 | 137 | 1,12 |
| DSM 21062 | 53 | <,5 | 30 | 53 | 89 | 5650 | 1824 | <,5 | 7,70 |

### Example 3: Strain bulkiness and thickness.

### Strain bulkiness

The strains were cultivated as described above. The optical density of the individual strains was adjusted to 4.04 (OD at 600 nm), i.e. adjusted to same amount of bacterial biomass. Ten ml of the strains DSM 21062, CHCC8773 and CHCC8818 were subsequently centrifuged at 2400 x g for 15 min in 15 ml conical tubes.

### Results

The height of the pellets was 16, 5 and 4 mm for the strains DSM 21062. CHCC8773 and CHCC8818, respectively (see figure 4). The bulkiness of DSM 21062 confirms that this strain produces high amounts of exopolysaccharide and possibly also capsular polysaccharide.

### Strain thickness

The strains were cultivated as described above. The optical density of the individual strains was adjusted to 4.04 (OD at 600 nm). The rheological measurements were performed on a Stresstech Rheometer (Rheologica AB, Lund, Sweden) using a standard C25 bob and cup geometry (2.5 cm in diameter). The samples were tempered to 13°C in a thermo chamber and analyzed at this temperature. The shear stress was measured at a shear rate of 300 s-1.

### Results

Although all strains have low viscosity, the strain DSM 21062 has a slightly increased viscosity compared to CHCC8773 and CHCC8818 (see table III and Figure 4).

**Table III: Shear stress and viscosity of DSM 21062, CHCC8773 and CHCC8818.**

| **Strain** | **Shear stress (Pa)** | **Viscosity (mPa s)** |
|---|---|---|
| DSM 21062 | 0.82 | |
| | | 2.7 |
| DSM 21062 | 0.83 | |
| | | 2.8 |
| CHCC8773 | 0.79 | 2.6 |
| CHCC8773 | 0.79 | 2.6 |
| CHCC8818 | 0.78 | 2.6 |
| CHCC8818 | 0.75 | 2.5 |

### Example 4: Inhibition of pathogen growth

Probiotic isolates (CHCC9555, CHCC9553, CHCC8773, CHCC8818. and DSM 21062) were cultivated in MRS medium for 3 days as described in example 1. Growth medium for the pathogens *Staphylococcus aureus* and *Listeria monocytogenes* was BHI-broth (Difco, BD Diagnostics, Sparks, MD, USA or Oxoid Limited Hampshire, UK) and for *Escherichia coli* and *Salmonella typhimurium* LB-broth (Difco, BD Diagnostics, Sparks, MD, USA or Oxoid Limited Hampshire, UK). The pathogen cultures were obtained from Oxoid Limited Hampshire, UK.

The conditioned media from the probiotic cultures were recovered by centrifugation. The conditioned media were pH adjusted to pH 6.5 - 6.7 and stored at 4°C until use. The pathogen cultures were inoculated from frozen glycerol stocks or lyophilized inoculation loops in the morning. When OD600 reached 0.2 or higher, pathogen cultures were added in triplicate to microtiter plates with or without probiotic conditioned media. The growth of the pathogens was monitored every 30 min by OD600 measurement in a microtiter plate reader. The slopes of the growth curves (1 - 3.5 hrs) were calculated (least squares method) and compared to express the inhibition by the supernatants. Positive controls (probiotic strains La-5 and CRL-431 both available from Chr. Hansen A/S, Hoersholm, Denmark), negative control (MRS pH 6.5) and acid-control (MRS pH 4.2) were included on every microtiter plate.

### Results

Results are expressed as the percentage, with the slope of the positive control (pathogen without any inhibiting supernatants) defined as 100%. While some of the strains have no effect on pathogen growth or even enhance pathogen growth, the inhibition of pathogen growth is superior for the strain DSM 21062 compared to the strains CHCC9555. CHCC9553, CHCC8773, and CHCC8818 (see table VI).

**Table VI: Growth of pathogenic bacteria in presence of conditioned media from probiotic cultures. No inhibition of growth is defined as 100%. (E. coli: Eschericia coli, S. aureus: Staphylococcus aureus. L. mono: Listeria monocytogenes, S. typh: Salmonella typhimurium).**

| **Strain** | **E. coli (%)** | **S. aureus (%)** | **L- mono. (%)** | **S. typh. (%)** |
|---|---|---|---|---|
| CHCC9555 | 101 | 80 | 86 | 80 |
| CHCC9553 | 85 | 76 | 74 | 81 |
| CHCC8773 | 97 | 75 | 41 | 97 |
| CHCC8818 | 112 | 86 | 61 | 108 |
| DSM 21062 | 61 | 43 | 60 | 73 |

### Example 5: Bile assay

The bile tolerance assay was performed essentially as described in Noriega et al. 2004 (37). Briefly, bacterial cultures were prepared as described in example 1. MRS agar plates supplemented with 0, 0.63, 0.125, 0.25, 0.5, 1.0, and 2.0% bile (bovine or porcine, Sigma B3883 and B8631, respectively) were prepared and 20 µl of the bacterial cultures were seeded onto the plates. After two days anaerobic incubation at 37°C the plates were examined for growth/no growth to determine minimal inhibitory concentration (MIC).

### Results

All strains have excellent tolerance to bovine and porcine bile (MIC > or = 2%) (see table IV), except CHCC8773 which displays poor to tolerance to bovine bile (MIC = 0.5%) and even less tolerance to porcine bile (0.25%). This finding may explain the poor performance on this strain (CHCC8773) in the colitis model (see example 1).

**Table V: Minimal inhibitory bile concentration. (MIC: Minimal inhibitory concentration).**

| **Strain** | **MIC Bovine bile (%)** | **MIC Porcine bile (%)** |
|---|---|---|
| CHCC9555 | 2 | >2 |
| CHCC9553 | >2 | >2 |
| CHCC8773 | 0.5 | 0.25 |
| CHCC8818 | 2 | >2 |
| DSM 21062 | 2 | >2 |

### Example 6: Bifidobacterium longum DSM 21062 strengthens tight junctions in vitro.

### Strains and culture conditions

Strains: *Bifidobacterium longum* strain (DSM 21062), *Bifidobacterium animalis* subsp. *lactis* strain BB-12® (DSM15954), *Lactobacillus acidophilus* strain La5 (DSM13241), and *Lactobacillus paracasei* subsp. *paracasei* strain CRL431, (ATCC 55544). *Bifidobacterium animalis* subsp. *lactis* strain BB-12® (DSM15954), *Lactobacillus acidophilus* strain La5 (DSM13241). and *Lactobacillus paracasei* subsp. *paracasei* strain CRL431, (ATCC 55544) are commercially available from Chr. Hansen A/S, 10-12 Boege Alle, DK-2970 Hoersholm, Denmark.

*Bifidobacterium animalis* subsp. *lactis* was grown at 37 °C in MRS broth (Difco. BD Dianostics, Sparks, MD, USA) + 0,05 % hydrochloride cysteine, using Anaerobic A incubation bags (Merck, Darmstadt, Germany). *Bifidobacterium longum* was grown at 37 °C in MRS broth (Difco. BD Dianostics, Sparks, MD, USA) + 0,05 % hydrochloride cysteine + 1 % sucrose, using Anaerobic A incubation bags (Merck, Darmstadt, Germany).

The lactobacilli were grown at 37 °C in MRS broth, using Anaerobic A incubation bags (Merck, Darmstadt, Germany).

### Cultivation of Caco-2 cells

Caco-2 cells (ATCC HTB-37, LGC Standards AB, Boras, Sweden) are derived from a human colorectal adenocarcinoma. This intestinal epithelial cell line is a model of ileocecal epithelial cells. The Caco-2 cells were grown in Dulbecco's *Modified Eagle Medium* with stable Glutamax-1 (Invitrogen, Carlsbad, CA) supplemented 20 % of fetal bovine serum (Hyclone, Logan, UT), 1,25 % Gentamicin/Amphotericin (Invitrogen, Carlsbad, CA), 1 % non-essential aminoacids (Invitrogen, Carlsbad, CA).

Caco-2 cells were cultured on sterile polyester membrane transwell-clear inserts (pore size: 0.4 µm; growth surface area: 0.33 cm²; membrane diameter: 6.5 mm; from Costar; Corning Incorporated Life Sciences, Lowell, MA). The cells were seeded at a density of approximately 60.000 cells/cm² and grown in 19-21 days with medium changes every second day. In every Transwell insert there was 600 µl growth medium on the basolateral side and 120 µl on the apical side.

### Co-incubation of bacterial cultures and Caco-2 cells.

Bacterial cultures in log-phase were diluted to 6.6x10⁶ bacteria/ml. To ensure survival of the bacterial cultures, the Caco-2 cell layer was washed twice in HBSS and incubated for 2 hours with gentamicin-free growth medium. Each bacterial strain (120 µl, *i.e.* 100 bacteria/cell) was incubated for 6 hours on the apical side of the Caco-2 cell cell layers in transwells (in triplicates). After 6 hours the cultures were removed and the cell layer washed twice with HBSS.

Sodium decanoate (C10) (Sigma-Aldrich, St. Louis, MO) was added (120 µl of an 8 mM solution in HBSS) to the cells to induced opening of the tight junctions. The C10 was left 15 min on the apical side of the Caco-2 cell layers in transwells before measurement of transepithelial electrical resistance (TEER). TEER was measured at 37°C in an Endohm 6 mm culture cup from World Precision Instruments, Stevenage, UK.

Two controls were included in the experiment. Both controls were incubated with growth for 6 hours simultaneously with the incubation of cultures. Thereafter one control was left with the growth medium while the other received C10 treatment for 15 minutes.

### Results

The TEER of the cell cultures was 1711 (+/- 83) Ohm x cm² for the untreated Caco-2 cell layer, while the TEER of cell layers challenged with C10 was reduced to 687 (+/- 94) Ohm x cm². Treatment of the cell layer with probiotic bacterial strains before the C10 challenge significantly decreased the TEER reduction for CRL 431 (855 (+/-46) Ohm x cm²) and La-5 (825 (+/-46) Ohm x cm²). In contrast to the bifidobacterium strain BB-12 that was unable to significantly improve the TEER of the cell layers, the bifidobacterium strain DSM21062 was the strain with the best capacity to decrease the reduction of TEER induced by C10 challenge. The TEER bifidobacterium strain DSM21062 was measured to 1071 (+/-22) Ohm x cm².

### Statistical analysis

Results were analyzed by the students t-test (Graphpad Prism software: http://www.graphpad.com). Differences were judged to be statistically significant when the p value was < 0.05.

### REFERENCES:

### Reference List

(1) Lebeer S, Ceuppens J, Geboes K et a/. Mechanisms of probiotic-host interaction with IBD as a case study: a role for exopolysaccharides? Commun Agric Appl Biol Sci 2007;72(1):41-5.
(2) Ruas-Madiedo P, Moreno JA, Salazar N et al. Screening of exopolysaccharide-producing Lactobacillus and Bifidobacterium strains isolated from the human intestinal microbiota. Appl Environ Microbiol 2007:73(13):4385-8.
(3) Ruas-Madiedo P, de los Reyes-Gavilan CG. Invited review: methods for the screening, isolation, and characterization of exopolysaccharides produced by lactic acid bacteria. J Dairy Sci 2005;88(3):843-56.
(4) Looijesteijn PJ, Trapet L, de Vries E et al. Physiological function of exopolysaccharides produced by Lactococcus lactis. Int J Food Microbiol 2001;64(1-2):71-80.
(5) Mao Y, Doyle MP, Chen J. Role of colanic acid exopolysaccharide in the survival of enterohaemorrhagic Escherichia coli O157:H7 in simulated gastrointestinal fluids. Lett Appl Microbiol 2006;42(6):642-7.
(6) Perdigon G, de Moreno dL, Valdez J et al. Role of yoghurt in the prevention of colon cancer. Eur J Clin Nutr 2002;56 Suppl 3:S65-S68.
(7) Chabot S, Yu H-L, De Léséleuc L et al. Exopolysaccharides from Lactobacillus rhamnosus RW-9595M stimulated TNF, IL-6, and IL-12 in human and mouse cultured immunocompetent cells, and IFN-g in mouse spleenocytes. Lait 2001;81:683-97.
(8) Kitazawa H, Harata T, Uemura J et al. Phosphate group requirement for mitogenic activation of lymphocytes by an extracellular phosphopolysaccharide from Lactobacillus delbrueckii ssp. bulgaricus. Int J Food Microbiol 1998;40(3):169-75.
(9) Ruas-Madiedo P, Gueimonde M, Margolles A et al. Exopolysaccharides produced by probiotic strains modify the adhesion of probiotics and enteropathogens to human intestinal mucus. J Food Prot 2006;69(8):2011-5.
(10) Ruas-Madiedo P, Gueimonde M, de los Reyes-Gavilan CG et al. Short communication: effect of exopolysaccharide isolated from "viili" on the adhesion of probiotics and pathogens to intestinal mucus. J Dairy Sci 2006;89(7):2355-8.
(11) Kodali VP, Sen R. Antioxidant and free radical scavenging activities of an exopolysaccharide from a probiotic bacterium. Biotechnol J 2008;3(2):245-51.
(12) Korakli M, Ganzle MG, Vogel RF. Metabolism by bifidobacteria and lactic acid bacteria of polysaccharides from wheat and rye, and exopolysaccharides produced by Lactobacillus sanfranciscensis. J Appl Microbiol 2002;92(5):958-65.
(13) Sengul N, Aslim B, Ucar G et al. Effects of exopolysaccharide-producing probiotic strains on experimental colitis in rats. Dis Colon Rectum 2006;49(2):250-8.
(14) Ewaschuk JB, Dieleman LA. Probiotics and prebiotics in chronic inflammatory bowel diseases. World J Gastroenterol 2006;12(37):5941-50.
(15) Mayer L. Mucosal immunity. Pediatrics 2003;111(6 Pt 3):1595-600.
(16) Rosenfeldt V, Benfeldt E, Valerius NH et al. Effect of probiotics on gastrointestinal symptoms and small intestinal permeability in children with atopic dermatitis. J Pediatr 2004;145(5):612-6.
(17) Mawe GM, Coates MD, Moses PL. Review article: intestinal serotonin signalling in irritable bowel syndrome. Aliment Pharmacol Ther2006;23(8):1067-76.
(18) Kajander K, Krogius-Kurikka L, Rinttila T et al. Effects of multispecies probiotic supplementation on intestinal microbiota in irritable bowel syndrome. Aliment Pharmacol Ther 2007;26(3):463-73.
(19) Kajander K, Myllyluoma E, Rajilic-Stojanovic M et al. Clinical trial: multispecies probiotic supplementation alleviates the symptoms of irritable bowel syndrome and stabilizes intestinal microbiota. Aliment Pharmacol Ther 2008;27(1):48-57.
(20) Rolfe RD. The role of probiotic cultures in the control of gastrointestinal health. J Nutr 2000;130(2S Suppl):396S-402S.
(21) O'Hara AM, Shanahan F. The gut flora as a forgotten organ. EMBO Rep 2006;7(7):688-93.
(22) Shanahan F. Physiological basis for novel drug therapies used to treat the inflammatory bowel diseases I. Pathophysiological basis and prospects for probiotic therapy in inflammatory bowel disease. Am J Physiol Gastrointest Liver Physiol 2005;288(3):G417-G421.
(23) Carroll MC, Prodeus AP. Linkages of innate and adaptive immunity. Curr Opin Immunol 1998;10(1):36-40.
(24) Diebold SS, Plank C, Cotten M et al. Mannose receptor-mediated gene delivery into antigen presenting dendritic cells. Somat Cell Mol Genet 2002;27(1-6):65-74.
(25) Stahl PD, Ezekowitz RA. The mannose receptor is a pattern recognition receptor involved in host defense. Curr Opin Immunol 1998;10(1):50-5.
(26) Keler T, Ramakrishna V, Fanger MW. Mannose receptor-targeted vaccines. Expert Opin Biol Ther 2004;4(12):1953-62.
(27) Hapfelmeier S, Muller AJ, Stecher B et al. Microbe sampling by mucosal dendritic cells is a discrete, MyD88-independent step in DeltainvG S. Typhimurium colitis. J Exp Med 2008;205(2):437-50.
(28) Li X, Jiao LL, Zhang X et al. Anti-tumor and immunomodulating activities of proteoglycans from mycelium of Phellinus nigricans and culture medium. Int Immunopharmacol 2008;8(6):909-15.
(29) Pang X, Chen Z, Gao X et al. Potential of a novel polysaccharide preparation (GLPP) from Anhui-grown Ganoderma lucidum in tumor treatment and immunostimulation. J Food Sci 2007;72(6):S435-S442.
(30) Lahtinen SJ, Jalonen L, Ouwehand AC et al. Specific Bifidobacterium strains isolated from elderly subjects inhibit growth of Staphylococcus aureus. Int J Food Microbiol 2007;117(1):125-8.
(31) Lachman LLHAKJ. The Theory and Practice of Industrial Pharmacy. 3rd ed. Philadelphia: Lea and Febiger Inc., 1986.
(32) Laulund S. Commercial Aspects of Formulation, Production and Marketing of Probiotic Products. In: Gibson SAW, editor. Human Health: The Contribution of Microorganisms. London: Springer-Verlag, 1994: 159-73.
(33) Foligne B, Nutten S, Steidler L et al. Recommendations for improved use of the murine TNBS-induced colitis model in evaluating anti-inflammatory properties of lactic acid bacteria: technical and microbiological aspects. Dig Dis Sci 2006;51(2):390-400.
(34) Wallace JL, MacNaughton WK, Morris GP et al. Inhibition of leukotriene synthesis markedly accelerates healing in a rat model of inflammatory bowel disease. Gastroenterology 1989;96(1):29-36.
(35) Bradley PP, Priebat DA, Christensen RD et al. Measurement of cutaneous inflammation: estimation of neutrophil content with an enzyme marker. J Invest Dermatol 1982;78(3):206-9.
(36) Yang Z, Huttunen E, Staaf M et al. Separation, purification and characterisation of extracellular polysaccharides produced by slime-forming Lactococcus lactis ssp. cremoris strains. International Dairy Journal 1999;9(9):631-8.
(37) Noriega L, Gueimonde M, Sanchez B et al. Effect of the adaptation to high bile salts concentrations on glycosidic activity, survival at low PH and cross-resistance to bile salts in Bifidobacterium. Int J Food Microbiol 2004;94(1):79-86.

### Regarding Deposited Microbial Organisms [EXPERT SOLUTION]

For all deposited microbial organisms mentioned in the present patent application and which not are in collections open to the public the so-called expert solution is requested.

In respect to those designations in which a European Patent is sought a sample of the deposited microorganism will be made available until the publication of the mention of the grant of the European patent or until the date on which application has been refused or withdrawn or is deemed to be withdrawn, only by the issue of such a sample to an expert nominated by the person requesting the sample, and approved either i) by the Applicant and/or ii) by the European Patent Office, whichever applies. (Rule 32 EPC-2000).

## Claims

1. A strain of *Bifidobacterium longum* bacterial cells, which is useful as probioticum, tolerates bile salts, produces a high amount of exopolysaccharides (EPS), and possesses anti-inflammatory effects in a trinitrobenzene sulfonate (TNBS)-induced colitis model in mice, **characterized in that** the strain is the *Bifidobacterium longum* strain with the registration number DSM 21062 or a mutant strain thereof, wherein the mutant strain is obtained by using the deposited strain as starting material, and wherein the mutant has retained or further improved the anti-inflammatory effects, the bile tolerance and/or the EPS expression that characterize DSM 21062.

2. The strain according to claim 1, wherein co-incubating an in vitro cultured layer of Caco-2 cells (ATCC HTB-37) with said bacterial strain results in a statistically significant strengthening of the tight junctions of the Caco-2 layer of cells measured as the transepithelial electrical resistance of the Caco-2 layer of cells after the Caco-2 layer of cells has been exposured to the tight junction disruptive agent sodium decanoate.

3. The strain according to claim 1 or 2, wherein the strain furthermore is able to inhibit the growth of pathogenic bacterial strains.

4. The strain according to any of claims 1 to 3, wherein the strain produces EPS which are relatively rich in mannose residues, measured as a Glu-N:Mannose-ratio of 40 or more.

5. The strain according to any of claims 1 to 4, wherein the strain produces EPS which are relatively rich in glucose residues, measured as a Glu-N:Glucose - ratio of 50 or more.

6. Use of a composition comprising a strain of bacterial cells according to any of claims 1-5 or a fraction of said cells for the preparation of a medicament.

7. Use of a composition comprising a strain of bacterial cells according to any of claims 1 to 5 or a fraction of said cells for the preparation of a medicament for the treatment of inflammatory conditions in the gastro-intestinal tract of a mammal.

8. A strain of bacterial cells according to any of claims 1 to 5 or a fraction of said cells for use in the treatment of inflammatory conditions in the gastro-intestinal tract of a mammal.

9. A human or pet food composition comprising at least one strain of bacterial cells according to any of claims 1 to 5.

10. A starter culture composition comprising the bacterial cells of any of claims 1 to 5, preferably wherein the starter culture composition is having a concentration of viable cells, which is in the range of 10⁴ to 10¹² CFU per gram of the composition.

11. A method of manufacturing a food or feed product comprising adding a starter culture composition according to claim 10 to a food or feed product starting material and keeping the thus inoculated starting material under conditions where the lactic acid bacterium is metabolically active.

## Patentansprüche

1. Bifidobacterium-longum-Bakterienzellenstamm, der nützlich als Probiotikum ist, Gallensalze toleriert, eine hohe Menge an Exopolysacchariden (EPS) produziert und entzündungshemmend bei einem Trinitrobenzolsulfonat (TNBS)-induzierten Dickdarmentzündungsmodell bei Mäusen wirkt, **gekennzeichnet dadurch, dass** der Stamm der Bifidobacterium-longum-Stamm mit der Hinterlegungsnummer DSM 21062 oder ein Mutantenstamm davon ist, wobei der Mutantenstamm durch Verwendung des hinterlegten Stamms als Ausgangsmaterial erhalten wird, und wobei der Mutant die entzündungshemmende Wirkung, die Gallentoleranz und/oder die EPS-Expression von DSM 21062 beibehalten oder weiter verbessert hat.

2. Stamm nach Anspruch 1, wobei die Koinkubation einer in vitro kultivierten Schicht Caco-2- ellen (ATCC HTB-37) mit besagtem Bakterienstamm zu einer statistisch signifikanten Verstärkung der tight junctions des Caco-2-Zellschicht führt, gemessen als die transepitheliale elektrische Resistenz der Caco-2-Zellschicht, nachdem die Caco-2-Zellschicht dem tight junctions auflösenden Natriumdecanoat ausgesetzt wurde.

3. Stamm nach Anspruch 1 oder 2, wobei der Stamm darüberhinaus das Wachstum von krankheitserregenden Bakterienstämmen hemmen kann.

4. Stamm nach einem beliebigen der Ansprüche 1 bis 3, wobei der Stamm EPS produziert, die verhältnismäßig reich an Mannoserückständen sind, gemessen als Glu-N:Mannose-Verhältnis von 40 oder mehr.

5. Stamm nach einem beliebigen der Ansprüche 1 bis 4, wobei der Stamm EPS produziert, die verhältnismäßig reich an Traubenzuckerrückständen sind, gemessen als Glu-N:Glucose-Verhältnis von 50 oder mehr.

6. Verwendung einer Zusammensetzung mit einem Bakterienzellenstamm nach einem beliebigen der Ansprüche 1-5 oder einem Teil dieser Zellen zur Herstellung eines Medikaments.

7. Verwendung einer Zusammensetzung mit einem Bakterienzellenstamm nach einem beliebigen der Ansprüche 1 bis 5 oder einem Teil dieser Zellen zur Herstellung eines Medikaments zur Behandlung von Entzündungen im Magen-Darm-Trakt eines Säugetiers.

8. Bakterienzellenstamm nach einem beliebigen der Ansprüche 1 bis 5 oder ein Teil dieser Zellen zum Einsatz bei der Behandlung von Entzündungen im Magen-Darm-Trakt eines Säugetiers.

9. Speise- oder Tierfutter-Zusammensetzung mit mindestens einem Bakterienzellenstamm nach einem beliebigen der Ansprüche 1 bis 5.

10. Starterkultur-Zusammensetzung mit den Bakterienzellen nach einem der Ansprüche 1 bis 5, wobei vorzugsweise die Starterkultur-Zusammensetzung eine Konzentration von lebensfähigen Zellen im Bereich von 10⁴ bis 10¹² CFU pro Gramm der Zusammensetzung aufweist.

11. Verfahren zur Herstellung eines Lebens- oder Futtermittels unter Hinzufügung einer Starterkultur-Zusammensetzung nach Anspruch 10 zu einem Ausgangsmaterial für ein Lebens- oder Futtermittel und Halten des so inokulierten Ausgangsmaterials unter Bedingungen, bei denen die Milchsäurebakterie metabolisch aktiv ist.

## Revendications

1. Souche de cellules bactériennes de *Bifidobacterium longum* qui est utile comme probiotique, tolère les sels biliaires, produit une grande quantité d'exopolysaccharides (EPS) et possède des effets anti-inflammatoires dans une forme de colite chez les souris, induite par le sulfonate de trinitrobenzène (TNBS), **caractérisée en ce que** la souche est la souche de *Bifidobacterium longum* ayant le numéro d'immatriculation DSM 21062 ou une souche mutante de celle-ci, où la souche mutante est obtenue en utilisant la souche déposée comme matière première, et où le mutant a retenu ou amélioré ultérieurement les effets anti-inflammatoires, la tolérance des sels biliaires et/ou l'expression d'EPS qui caractérisent DSM 21062.

2. La souche selon la revendication 1, où le fait de co-incuber une couche cultivée in vitro de cellules Caco-2 (ATCC HTB-37) avec ladite souche bactérienne comporte une consolidation statistiquement significative des jonctions serrées de la couche de cellules Caco-2 mesurée comme la résistance électrique transépithéliale de la couche de cellules Caco-2 après que la couche de cellules Caco-2 a été exposée à l'agent dissolvant les jonctions serrées décanoate de sodium.

3. La souche selon la revendication 1 ou 2, où la souche peut en outre inhiber la croissance de souches bactériennes pathogènes.

4. La souche selon l'une quelconque des revendications 1 à 3, où la souche produit des EPS qui sont relativement riches en résidus de mannose, mesurée comme un rapport de Glu-N:mannose de 40 ou plus.

5. La souche selon l'une quelconque des revendications 1 à 4, où la souche produit des EPS qui sont relativement riches en résidus de glucose, mesurée comme un rapport de Glu-N:Glucose de 50 ou plus.

6. Utilisation d'une composition comprenant une souche de cellules bactériennes selon l'une quelconque des revendications 1-5 ou une fraction desdites cellules pour la préparation d'un médicament.

7. Utilisation d'une composition comprenant une souche de cellules bactériennes selon l'une quelconque des revendications 1 à 5 ou une fraction desdites cellules pour la préparation d'un médicament pour le traitement de conditions inflammatoires dans le tractus gastro-intestinal d'un mammifère.

8. Souche de cellules bactériennes selon l'une quelconque des revendications 1 à 5 ou une fraction desdites cellules pour l'utilisation dans le traitement de conditions inflammatoires dans le tractus gastro-intestinal d'un mammifère.

9. Composition alimentaire destinée aux humains ou aux animaux domestiques comprenant au moins une souche de cellules bactériennes selon l'une quelconque des revendications 1 à 5.

10. Composition de culture starter comprenant les cellules bactériennes de l'une quelconque des revendications 1 à 5, où de préférence la composition de culture starter a une concentration de cellules viables de l'ordre de 10⁴ à 10¹² CFU par gramme de la composition.

11. Méthode de fabrication d'un produit alimentaire ou aliment pour animaux comprenant l'addition d'une composition de culture starter selon la revendication 10 à une matière première de produit alimentaire ou aliment pour animaux en gardant la matière première ainsi inoculée dans des conditions où la bactérie d'acide lactique est métaboliquement active.
